# EUROPEAN PATENT APPLICATION

(11) **EP 4 596 680 A1**
(43) Date of publication of application: **06.08.2025**
(21) Application number: 23870900.0
(22) Date of filing: 27.09.2023
(51) Int. Cl.: C12N 5/0783, C12N 5/0786, C12N 5/10, A61K 39/00, A61P 35/00

(54) **IDENTIFICATION, IN VITRO AMPLIFICATION, AND APPLICATION METHOD OF MEMORY CD8 T CELLS SPECIFIC FOR TUMOR ANTIGEN**

(30) Priority: 30.09.2022 CN 202211211371
(71) Applicant: Beijing Changping Laboratory, Changping District Beijing 102206 (CN)
(72) Inventor: HUANG, Qizhao, Beijing 102206 (CN)
(74) Representative: Meissner Bolte Partnerschaft mbB
(86) International application number: PCT/CN2023/121922
(87) International publication number: WO 2024/067673

(57) **Abstract**

The present application provides identification, *in vitro* amplification, and application method of memory CD8 T cells specific for an antigen in a solid tumor-draining lymph node.

## Description

### Technical Field

The present invention belongs to the field of T cell anti-tumor immune response and tumor immune-therapy, and in particular, relates to identification, *in vitro* amplification and application method of a new type of tumor antigen specific memory CD8 T cells in draining lymph nodes of solid tumor.

### Background Art

Malignant tumors are major diseases that seriously endanger public health in China. According to statistics released by the National Cancer Center, in 2015 alone, there were 3.929 million new cancer cases and 2.338 million deaths in China, both of which ranked first globally. Human's immune system can actively identify tumor antigens, exert the function of immune surveillance, and eliminate cancerous cells at an early stage. However, in certain conditions, tumor cells can escape human's immune surveillance through a variety of immune escape strategies, form primary tumors, and establish an immunosuppressive tumor microenvironment that inhibits anti-tumor immune responses and promotes tumor progression.

The anti-tumor immune response mediated by antigen-specific CD8 T cells is the main way for the mammals to combat tumorigenesis. CD8 T cells recognize the tumor antigen peptide-MHC I complex expressed on the surface of tumor cells through their T cell receptors (TCRs), specifically kill the tumor cells, and thus play key roles in anti-tumor immunity. However, once escaping immune surveillance through immunoediting, tumor cells will rapidly proliferate, form tumor tissues, and establish an immunosuppressive microenvironment. In this case, the persistence of tumor antigens and immunosuppressive molecules (such as PD-L1, CD48, Galectin-1, IDO, and IL-10, etc.) in tumor tissues continuously stimulate tumor-specific CD8 T cells through TCR and co-inhibitory signaling pathways, ultimately leading to their differentiation into terminally exhausted CD8 T cells (Exhausted T cell, T_{EX}). The concepts corresponding to exhausted T cells are effector T cells (effector T cells, Teff) and memory T cells (memory T cells, Tmem). Effector T cells have strong effector function, which can secrete functional cytokines (IFN-γ, TNF-α, etc.), and directly kill virus-infected target cells or cancer cells through the perforin-granzyme pathway. Memory T cells can maintain for a long time, and when they are encountered with cognate viruses or tumor antigens again, they can rapidly proliferate, and eliminate virus-infected or cancer cells quickly and efficiently. In contrast, exhausted CD8 T cells gradually lose their function of cytokine secretion and cytotoxicity, becoming prone to apoptosis and losing the potential to differentiate into memory cells, thus losing the ability to control tumor growth and progression at the population level. Therefore, in-depth research into the mechanism underlying CD8 T cell exhaustion in the tumor environment at the cellular and molecular levels may provide new insights for reversing exhaustion and improving tumor immunotherapy, which possesses important theoretical significance and clinical value.

At present, the strategy for overcoming CD8 T cell exhaustion is mainly to block inhibitory receptor signaling pathways. Immune checkpoint blockade therapy, represented by PD-1/PD-L1 blocking antibodies, has achieved good results in the treatment of malignant melanoma, non-small cell lung cancer and renal cancer. The median duration of response is about 2-3 times that of traditional chemotherapy, but the over-all patient response rate only reaches about 30%. In addition, clinical studies have found that patients who respond in the initial stage may develop drug resistance or relapse in the later stage, and only a very small number of patients (<5%) can be clinically cured.

At present, most opinions in the field believe that progenitor exhausted T cells (T_{PEX}) infiltrating tumor tissues are the critical cell subset that responds to PD-1 immune checkpoint therapy. However, this cell subset accounts for a low proportion of infiltrating tumor-specific CD8 T cells and has limited proliferative potential, which makes it difficult to explain the significant anti-tumor effect of PD-1 immune checkpoint blockade therapy. In addition, studies have shown that each subset of exhausted CD8 T cells infiltrating tumor tissues presents relatively steady epigenetic and transcriptional states, and PD-1 immune checkpoint blockade is difficult to change the steady epigenetic characteristics. Further improving the response rate and sustained effect of PD-1 immune checkpoint blockade therapy is currently a major challenge in the field of tumor immunotherapy. One of the keys to future breakthroughs may lie in clarifying and identifying new key CD8 T cell subsets that respond to PD-1 immunotherapy, and elucidating the molecular regulatory mechanism of their responses.

### Contents of the present invention

The present invention provides a new type of tumor antigen specific memory CD8 T cells in draining lymph nodes (TdLN) of solid tumor, as well as their identification, amplification and clinical application. Based on flow cytometry, the new type of non-exhausted tumor-specific CD8 T cells with memory cell characteristics that are enriched in mouse tumor draining lymph nodes was identified. This unique cell subset highly expresses memory T cell surface molecules CD62L, CD127, CD122 and CCR7, and lacks the expression of co-inhibitory receptors Tim3, LAG3, CD39 and 2B4, which distinguishes it from precursor exhausted cells and other exhausted cell subsets infiltrating in tumors; expresses CD44 (human-derived cells are CD45RA⁻CD45RO⁺), which distinguishes it from naive CD8 T cells; highly expresses transcription factor TCF-1, which distinguishes it from effector CD8 T cells; and expresses low levels of PD-1, which distinguishes it from classical memory CD8 T cells. This new cell subset is essential for the continuous replenishment and maintenance of the exhausted CD8 T cell subsets infiltrating in tumor tissues (Tumor infiltrating T cells, TILs). More importantly, this cell subset is the key cell subset that continuously responds to tumor PD-1/PD-L1 immune checkpoint blockade therapy.

In one aspect, the present invention provides an antigen-specific memory CD8 T cell derived from tumor-draining lymph nodes (hereinafter referred to as "the cell of the present invention" or "the CD8 T cell of the present invention" or "the novel cell population of the present invention"), characterized in that it is a cell having the following marker combination: an activated CD62L⁺PD1^{Low/dim}Tim3-CD8⁺ T cell.

In a preferred embodiment, the cell of the present invention is:
for mouse tumor source,
a CD44⁺CD62L⁺CD127⁺CD122⁺CCR7⁺PD1^{Low/dim}Tim3⁻CD39⁻LAG3⁻CD8⁺ T cell;
for human tumor source,
a CD3⁺CD4⁻CD45RA⁻CD45RO⁺CD62L⁺CD127⁺CD122⁺CCR7⁺PD1^{Low/dim}Tim3⁻CD39⁻ LAG3⁻CD8⁺ T.

In one aspect, the novel cell population of the present invention is characterized by high expression of CD122, CD62L, CD127, CCR7, lack of expression of 2B4, LAG3, Tim3, CD39, and low expression of inhibitory receptor PD-1; preferably, the novel cell population of the present invention highly expresses transcription factor TCF-1 and can continuously respond to PD-1/PD-L1 immune checkpoint blockade therapy.

In another aspect, the present invention provides the use of an antigen-specific memory CD8 T cell isolated from a tumor-draining lymph node in combating tumor. In a specific embodiment, the present invention provides the use of the T cell in an adoptive cell transfer therapy for inhibiting tumor growth, that is, by injecting/re-infusing the enriched or in-vitro expanded autologous or non-autologous T cells into a patient. In a preferred embodiment, the tumor is selected from melanoma and ovarian cancer. In a specific embodiment, the T cell is co-administered with PD-1/PD-L1 immune checkpoint blockade therapy, preferably, the PD-1/PD-L1 immune checkpoint blockade therapy is anti-PD-L1 or PD-1 antibody.

In another aspect, the present invention provides a method for isolating the T cell of the present invention from a tumor-draining lymph node, comprising the following steps:
a. obtaining a single cell suspension from mouse or human solid tumor-draining lymph node;
b. sorting an activated CD62L⁺PD1^{Low/dim}Tim3⁻CD8⁺ T cell from the above single cell suspension,

preferably, for the mouse, sorting a CD44⁺CD62L⁺CD127⁺CD122⁺CCR7⁺PD1^{Low/dim}Tim3⁻ CD39⁻LAG3⁻CD8⁺ T cell from the single cell suspension;
for the human, sorting a CD3⁺CD4⁻CD45RA⁻CD45RO⁺CD62L⁺CD127⁺CD122⁺CCR7⁺ PD1^{Low/dim}Tim3⁻CD39⁻LAG3⁻CD8⁺ T cell from the single cell suspension;
preferably, the T cell is obtained by flow cytometry or magnetic bead sorting; and
c. performing *in vitro* amplification and culture in T cell culture medium containing CD3/28 antibodies, and interleukin 2, interleukin 7 and interleukin 15 at low dose.

The purpose of the present invention is achieved by the following key technical solutions:
(1) the immunophenotype of antigen-specific non-exhausted memory CD8 T cell is identified in tumor-draining lymph nodes by using flow cytometry analysis, combined with a mouse tumor model and human ovarian cancer samples;
(2) a non-exhausted activated CD8 T cell subset with high expression of memory cell-related molecules and low expression of PD-1 is sorted out from tumor-draining lymph nodes by using flow cytometry, and the number of this cell is further increased under optimized T cell *in-vitro* culture conditions to prepare for subsequent treatment;
(3) a mouse tumor is treated by re-infusing this cell population alone or in combination with PD-1/PD-L1 immune checkpoint blockade therapy.

By using the method described in the present invention, the draining lymph node tumor antigen-specific memory CD8 T cells (Tumor specific memory CD8 T cells, T_{TSM}) can be obtained accurately and effectively. Compared with the exhausted precursor cells and tumor-infiltrating lymphocytes (TILs), T_{TSM} cells have an antigen-independent homeostatic proliferation ability and has a strong potential of rapid proliferation when it contacts the same tumor antigen again. In addition, each exhausted CD8 T cell subset (exhausted precursor cells and terminal exhausted cells) infiltrating in tumors depends on the replenishment of this cell population, and the adoptive transfer of this cell population can inhibit tumor growth. In addition, compared with the exhausted precursor cells, this cell population is a key cell subset that responds to PD-1/PD-L1 immune checkpoint blockade therapy, and shows a significant synergistic effect with PD-1/PD-L1 immune checkpoint blockade therapy. The present invention effectively improves the efficacy of tumor immunotherapy and provides a new direction and inspiration for related research in this field.

### Brief Description of the Drawings

Figure 1 shows the establishment of mouse subcutaneous transplant tumor model and the flow cytometry analysis strategy.
Figure 2 shows the immunophenotypic analysis of tumor-draining lymph node antigen-specific memory CD8 T cells.
Figure 3 shows the verification of the memory phenotype of tumor-draining lymph node antigen-specific memory CD8 T cells.
Figure 4 shows the effective control of tumor growth by tumor-draining lymph node antigen-specific memory CD8 T cells.
Figure 5 shows the important role of tumor-draining lymph node antigen-specific memory CD8 T cells in immune checkpoint blockade therapy and the synergistic effect with PD-L1 immunotherapy.
Figure 6 shows the presence of antigen-specific memory CD8 T cells in human ovarian cancer draining lymph nodes.

### Specific Models for Carrying Out the present invention

The technical solution of the present application is described below in detail in conjunction with the examples, but those skilled in the art will understand that the following examples are only used to illustrate the present application, rather than to limit the scope of the present application. According to the following detailed description of the preferred examples, the various objects and advantages of the present application will become apparent to those skilled in the art.

### Example 1: Establishment of transplanted tumor model for isolating antigen-specific memory CD8 T cells in mouse tumor-draining lymph nodes

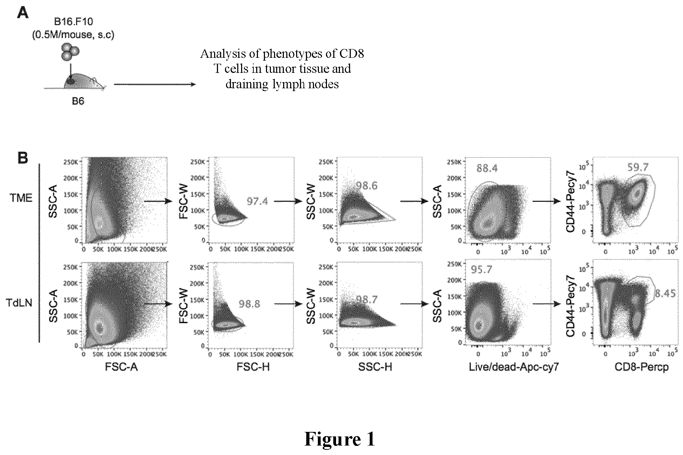

1. Preparation of tumor cell lines: In this example, mouse melanoma cell line B16.F10 was used to establish a mouse subcutaneous transplanted tumor model. The cell line was passaged and cultured *in vitro* using DMEM medium containing 10% fetal bovine serum (FBS), 1% penicillin-streptomycin and 2 mM L-glutamine.
2. Tumor cell inoculation: When the fusion density of mouse melanoma B16 cells reached about 80% to 90%, the cells were digested, and counted, and then the concentration of the tumor cell suspension was adjusted to 5×10⁶ cells/mL with 1×PBS buffer; 0.1 mL of the tumor cell suspension was taken using a 1 mL BD insulin syringe; the skin between the mouse's ears and back was held with one hand to fully expose the mouse's abdomen, and the skin of abdomen was cleared with 75% alcohol cotton balls; the syringe was held with the other hand, and the needle was inserted horizontally (the angle to the mouse's abdominal plane was about 0-15°) into the skin of the mouse's groin, and the tumor cell suspension was injected subcutaneously to form a small skin mound by the negative pressure of back suction. After the injection was completed, the needle was quickly withdrawn in parallel manner to prevent the cell suspension from flowing out.
3. The tumor growth was observed 8-10 days after the cell injection. Usually, it could be seen that the tumor grew to a size of a soybean in about 8 days. After that, the tumor size was measured by vernier caliper and recorded every two days (the tumor volume calculation formula was: tumor volume = 1/2 length × width², unit: mm³). The observation endpoint was determined according to the specific experimental requirements (Figure 1A). From an ethical point of view, the mouse was judged dead when the tumor volume reached 2000 mm³.

### Example 2: Isolation, acquisition and immunophenotypic identification of tumor-draining lymph node antigen-specific memory CD8 T cells

1. After the mouse was sacrificed, its inguinal subcutaneous lymph nodes that were usually located at the intersection of three superficial subcutaneous blood vessels in the lower abdominal wall were carefully separated; the lymph nodes were carefully ground with frosted area of a slide, and then the grinding area was rinsed with RPMI-1640 culture medium containing 10% fetal bovine serum, the above operation was repeated several times, and then the cell suspension was collected, and centrifuged at 4°C for 6 minutes, in which the centrifugal force was set at 400g. After centrifugation, the cells were resuspended with sterile PBS, and the cell pellets were gently pipetted several times; after centrifugation again, the cells were resuspended with a certain volume of the above culture medium, and a single cell suspension was obtained for later use.
2. Preferably: at the same time, the subcutaneous tumor tissue was separated, and ground thoroughly, and the lymphocytes infiltrating tumor tissue as a control were separated by density gradient centrifugation. The separation solution Percoll had the following density gradient: 44% for the upper layer and 67% for the lower layer. The centrifugation was performed at room temperature for 30 minutes, in which the centrifuge speed was set to reach 550 g.
3. Immunophenotypic analysis of tumor antigen-specific CD8 T cells
3.1 The cells separated from the tumor lymph nodes were counted to record the total number of cells, and then stained with fluorescent antibody. The steps for flow cytometry staining were referred to: Study on the role and mechanism of transcription factor TCF-1 in virus-specific T cell immune response. Huang Qizhao. 2018. Doctoral dissertation (published). In this example, we used Live/Dead (L/D) dye to distinguish dead cells, and to perform flow cytometry analysis, and the antigen-activated cells were labeled with CD3, CD8, and CD44. The number of antigen-specific CD8 T cells was determined based on their frequency in total cells and the total number of cells (Figure 1B).
3.2 Detection was performed on the expression of cell surface molecules, including inhibitory receptors such as PD-1, 2B4, LAG3, and Tim3; memory-related molecules such as CD122, CD62L, CD127, and CCR7, and other related cell surface molecules. The above surface molecules all could be quantitatively analyzed using flow cytometry;
3.3 Detection was performed on the intranuclear transcription factors, mainly including important transcription factors related to T cell differentiation, such as TCF-1, TOX, and Eomes.

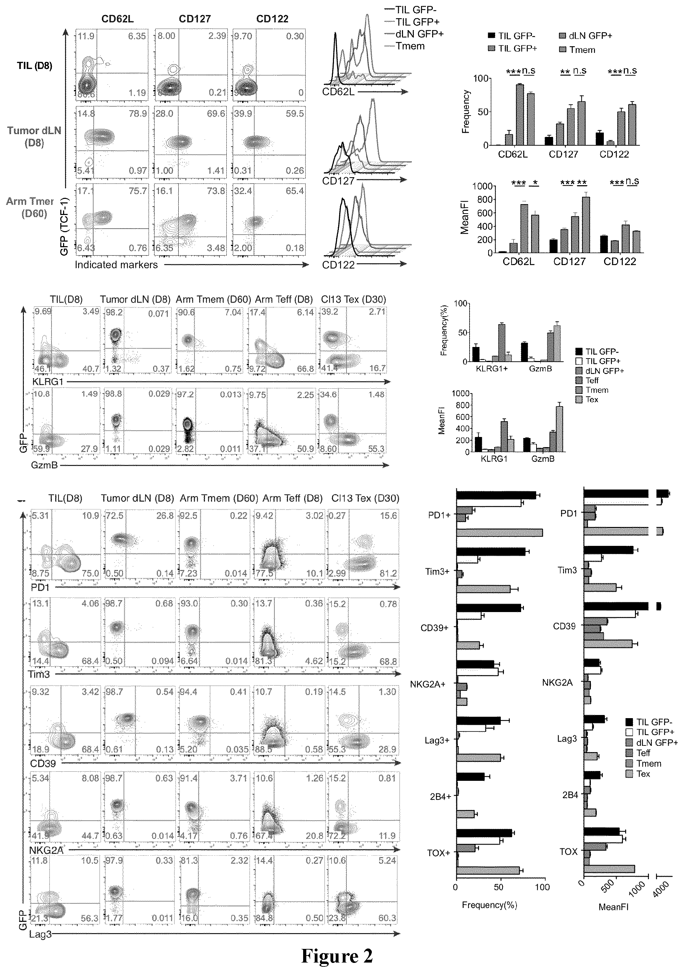

From the results, it could be seen that there was a population of antigen-specific CD8 T cells in the tumor-draining lymph nodes, which highly expressed memory-related molecules such as CD122, CD62L, CD127, and CCR7, and was lack of the expression of inhibitory receptors such as 2B4, LAG3, Tim3, and CD39; at the same time, this population of cells highly expressed memory cell-related transcription factor TCF-1 (represented by GFP) (see, Figure 2), suggesting that this population of cells had memory cell characteristics.

### Example 3: Phenotypic verification of tumor-draining lymph node antigen-specific memory CD8 T cells

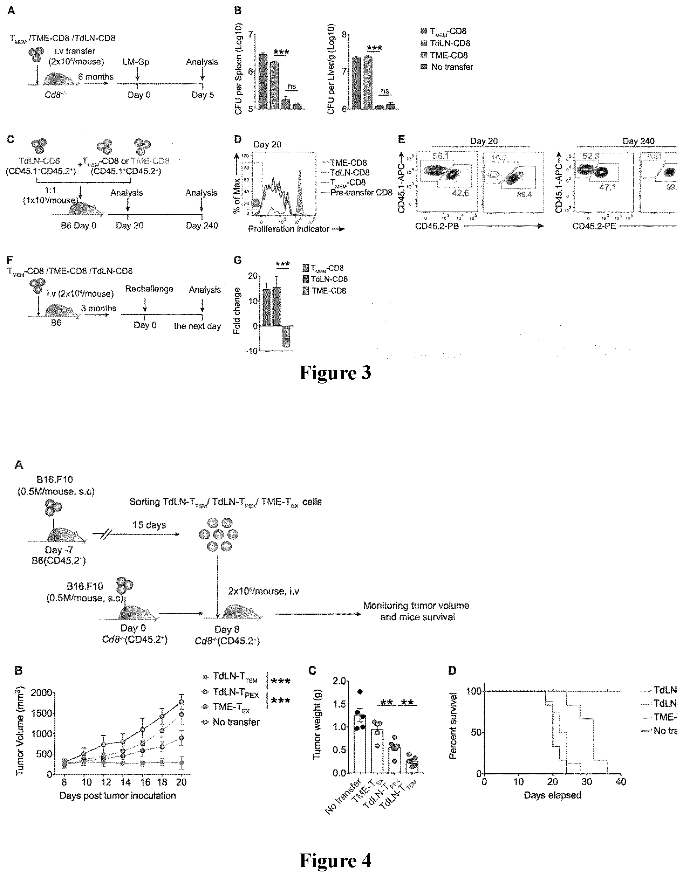

1. Sorting of target cell subset by flow cytometry technology: It was determined in Example 2 that the tumor-draining lymph node antigen-specific memory CD8 T cells could highly express memory T cell-related marker molecules, lowly express the exhausted cell-related marker molecule PD-1, and were lack of the expression of inhibitory receptors such as 2B4, LAG3, Tim3, and CD39. Therefore, we defined the draining lymph node antigen-specific memory CD8 T cell as CD44⁺CD62L⁺CD127⁺CD122⁺CCR7⁺PD1^{Low/dim}Tim3⁻CD39⁻LAG3⁻CD8⁺ T cell, and used this molecular marker to sort out the target cell subset from the mouse tumor-draining lymph nodes.
2. Verification of memory characteristics of target cell subset: The sorted target cell subset and a classical memory cell-labeling cell proliferation dye (Cell trace Violet) in acute infection model were mixed in the same amount and then injected back to naive recipient mice. The spleens of the recipient mice were taken at different time points to observe the long-term maintenance ability and homeostatic proliferation ability of the cells derived from the two sources (Figures 3C to 3E); in addition, the same number of cells derived from the two sources were mixed and then injected into naive recipient mice, and the same antigen stimulation was given again to observe the difference in the cell response ability of the memory cells respectively formed in the tumor-draining lymph nodes and the acute viral infection when encountering the same antigen (Figures 3A and 3B). The results showed that the draining lymph node antigen-specific memory cells were similar to the classical memory cells and capable of performing homeostatic proliferation in an antigen-independent manner (self-renew), and survived for a long time (Figures 3C to 3E); when stimulated again by the same antigen, they had better amplification ability (Figures 3A and 3B) and effector function than the antigen-specific cells infiltrating tumor tissues, thus better controlling *Listeria* infection expressing the same antigen (Figures 3F and 3G).

### Example 4: In vitro amplification and application of tumor-draining lymph node antigen-specific memory CD8 T cells

1 . *In vitro* amplification of target cell subset: The CD44⁺CD62L⁺CD127⁺CD122⁺CCR7⁺PD1^{Low/dim}Tim3⁻CD39⁻LAG3⁻CD8⁺ T cells isolated and obtained in Example 2 were cultured *in vitro* in an RPMI1640 medium containing 10% fetal bovine serum (FBS), to which 0.5µg/ml CD28 antibody, 1-5 ng/ml interleukin 2, and 20 ng/ml interleukin 7 and interleukin 15 (IL2/IL7/IL15) were added at the same time. After sorted, the target cells were counted and placed in a 24-well culture plate pre-coated with 2-5 µg/ml CD3 antibody, 1×10⁶ cells per well. After the cells were cultured for 2-3 days, the cell amplification was observed. When the amplification was good, the form of a large number of clone colonies could be seen. Then, the cells were transferred to a 12-well plate without CD3/28 antibodies. This method was used for continuous culture for 7-10 days and the cells were counted for the next experiment;
2. Effective control of tumor growth by tumor-draining lymph node antigen-specific memory CD8 T cells: When verifying the therapeutic effect of this population of cells from tumor-draining lymph nodes (B16 model), we selected antigen-specific CD8⁺ T cells infiltrating tumor tissue (tumor infiltrating lymphocytes, TIL) as a control. 2×10⁵ target cells were re-infused into tumor-bearing mice on days 8-10 of tumor inoculation, and then the tumor growth curve and the survival of tumor-bearing mice were recorded (Figure 4A). As shown in Figure 4, compared with the traditional tumor tissue infiltrating exhausted T cells (TME-TEx), the lymph node-derived memory cells of the present invention could more effectively control tumor growth and prolong the survival time of the tumor-bearing mice.

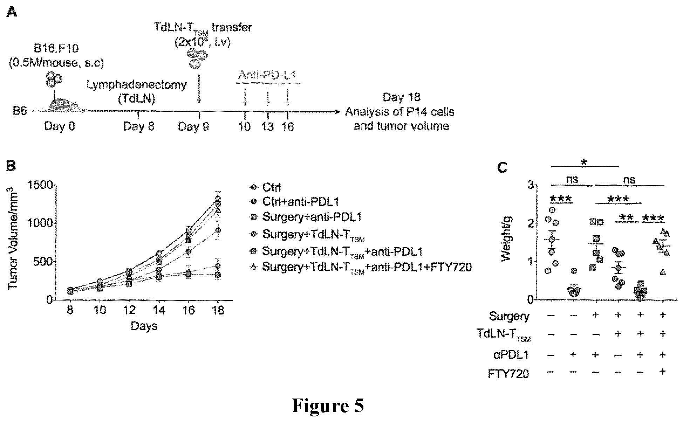

In addition, the tumor-bearing mice were subjected to the resection of bilateral draining lymph nodes on day 8, then re-infused with the lymph node antigen-specific memory cells, and administrated with PD-L1 blocking antibody (BioXCell, Cat#BE0101) or a control reagent (IgG) on days 10 and 13/16 of tumor inoculation, respectively, and at the same time, the group of cell re-infusion combined with anti-PD-L1 blocking antibody treatment was treated with FTY710 (Figure 5A). The results showed that after the lymph node resection, the anti-PD-L1 blocking antibody lost its tumor control effect. After the cells were re-infused and replenished, the anti-tumor effect of the blocking antibody could be effectively restored. If the lymphocyte migration inhibitor FTY-720 was used to block the migration of the cells from the lymph nodes to the periphery, the anti-PD-L1 blocking antibody also lost its tumor control effect (Figures 5B and 5C). The above results further suggested that the memory cells specific for antigens located in tumor-draining lymph nodes played an important role in tumor immunotherapy.

### Example 5: Isolation, acquisition and immunophenotypic identification of tumor-draining lymph node antigen-specific memory CD8 T cells of ovarian cancer patients

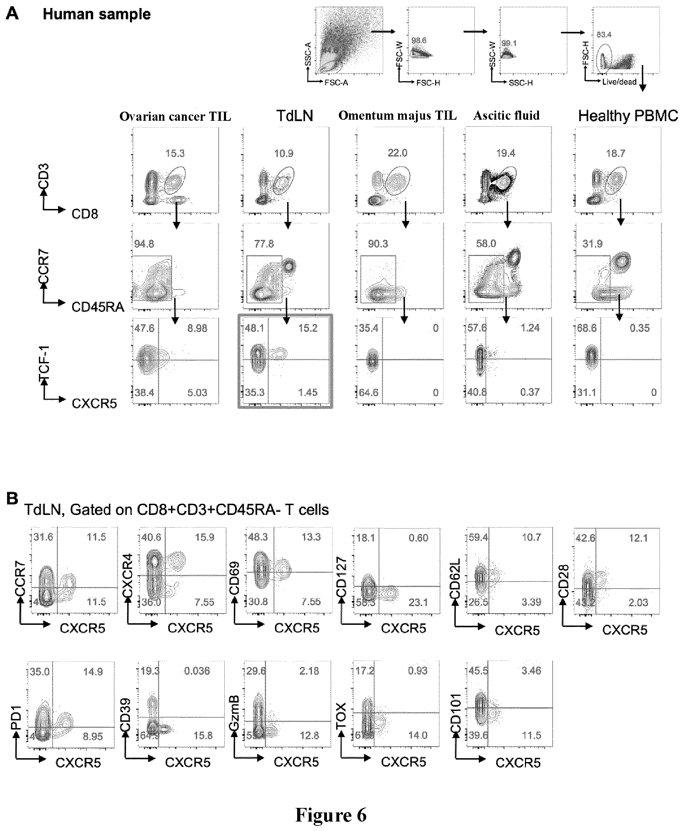

1. Screening of ovarian cancer patients: In this example, 2 ovarian cancer patients suitable for surgical resection were selected. Before surgery, informed consent forms were signed with the patients and approved by the ethics committee.
2. Processing of tumor specimens:
   (1) After the tumor tissue was resected surgically, the connective tissue attached to the surface was carefully removed, and the tumor tissue was cut into 1cm x 1cm tissue blocks, fully cut into pieces using ophthalmic scissors, and placed in 15ml centrifuge tubes. Then an appropriate volume (5-8 ml) of type I collagenase (Collagenase I) solution was added to each tube of tumor tissue, and digestion was performed in a shaking incubator at 37°C for 45min to 1h, in which the collagenase was dissolved in RPMI-1640 medium and had a working concentration of 1mg/ml.
   (2) After digestion, an equal volume of RPMI-1640 medium (R2) containing 2% FBS was added to each tube and mixed thoroughly by inversion to terminate the collagenase reaction. On a 70µm nylon filter, the tumor tissue was ground with the plastic end of syringe (or a 300 to 400 mesh stainless steel filter could be used instead).
   (3) After the cell suspension was centrifugated (2000rpm, 10min, room temperature), the supernatant was discarded. 8ml of 44% Percoll solution (volume ratio, diluted with RPMI) was added to each tube for resuspension. Then, capillary sampling needles (Pasteur tube) were inserted, and 4ml of 67% Percoll solution was slowly added. After standing for a while, it could be seen that the liquid was divided into two layers, i.e., red layer and white layer. The capillary glass needles were taken out, and centrifugation was performed at room temperature at 550g for 30min, and the speed-up and speed-down was set to zero (if possible, it was better that the above liquid was added to a 15ml round-bottomed bacterial tube or centrifuge tube and subjected to layered centrifugation).
   (4) After centrifugation, the flow tube was taken out, during which the removal process should be gentle to avoid damaging the layers. At this time, it could be seen that the liquid was divided into three layers, in which the middle layer was white and cloudy, which was the lymphocyte layer. First, a 1ml pipette was used to carefully discard the brown-yellow oil of the top layer. Then, the lymphocyte layer was slowly pipetted using a 1ml pipette that rotated during pipetting, added to a 15ml centrifuge tube with 3-5ml of R2 added in advance, mixed thoroughly, centrifuged (2000rpm, 10min, 4°C), resuspended, and counted.
   (5) After centrifugation, the supernatant was discarded, the lymphocytes were resuspended by adding a certain volume of RPMI-1640 culture medium (R10) containing 10% FBS for later use.
   (6) If the lymph nodes were small, they were ground directly on a 70µm nylon filter. If they were large, after being cut into pieces, they were ground again, counted, and frozen. If tumor cells metastasized to the lymph nodes, digestion with type I collagenase was also required after cutting into pieces.
3. Detection of immunophenotype of tumor antigen-specific CD8 T cells by flow cytometry
   (1) After the cells obtained in 2 was counted, 50 µl of staining buffer per 0.5×10⁶ to 1×10⁶ cells was added; and a flow cytometry working solution FACS buffer was prepared: PBS + 2%FBS + 0. 01%NaN₃
   (2) Fluorescent antibody labeling was performed according to the method described in Example 2.3, except that for human samples, the antigen-activated memory cells (corresponding to CD44 in mice) were labeled with CD45RA⁻CD45RO⁺.
   (3) From the results, it could be seen that antigen-specific memory CD8 T cells were also present in ovarian cancer tumor-draining lymph nodes. Similarly, the antigen-specific CD8 T cells were capable of highly expressing memory-related molecules such as CD122, CD62L, CD127, and CCR7, and were lack of expression of inhibitory receptors such as 2B4, LAG3, Tim3, and CD39; at the same time, this population of cells were capable of highly expressing the memory cell-related transcription factor TCF-1 (Figure 6). The above results further confirmed the presence of CD3⁺CD4⁻CD45RA⁻CD45RO⁺CD62L⁺CD127⁺CD122⁺CCR7⁺PD1^{Low/dim}Tim3⁻ CD39⁻LAG3⁻ CD8⁺ T, thereby providing a new theoretical basis for clinical immunotherapy of tumors.

## Claims

1. A tumor antigen-specific memory T cell derived from a tumor-draining lymph node, **characterized in that** it is a cell with the following marker combination:
an activated CD62L⁺PD1^{Low/dim}Tim3⁻CD8⁺ T cell.

2. The T cell according to claim 1, **characterized in that** it is a T cell with the following marker combination:
for mouse tumor source: a CD44⁺CD62L⁺CD127⁺CD122⁺CCR7⁺PD1^{Low/dim}Tim3⁻CD39-LAG3⁻CD8⁺ T cell;
for human tumor source: a CD3⁺CD4⁻CD45RA⁻CD45RO⁺CD62L⁺CD127⁺CD122⁺CCR7⁺ PD1^{Low/dim}Tim3⁻CD39⁻LAG3⁻CD8⁺ T cell.

3. The T cell according to claim 1 or 2, wherein the T cell is obtained by flow cytometry or magnetic bead sorting.

4. The T cell according to any one of claims 1 to 3, **characterized in that** it highly expresses CD122, CD62L, CD127, CCR7, lacks expressing 2B4, LAG3, Tim3, CD39, expresses inhibitory receptor PD-1 at a relatively low level, and highly expresses transcription factor TCF-1.

5. The T cell according to any one of claims 1 to 4, **characterized in that** it is capable of continuously responding to PD-1/PD-L1 immune checkpoint blockade therapy.

6. Use of the T cell according to any one of claims 1 to 5 in the manufacture of a medicament for inhibiting tumor growth, wherein preferably, the T cell inhibits tumor growth through adoptive cell transfer therapy; preferably, the tumor is selected from melanoma and ovarian cancer.

7. Use of a combination of the T cell according to any one of claims 1 to 5 and a PD-1/PD-L1 immune checkpoint blockade therapy in the manufacture of a medicament for inhibiting tumor growth, wherein preferably, the T cell inhibits tumor growth through adoptive cell transfer therapy; preferably, the tumor is selected from melanoma and ovarian cancer.

8. The use according to claim 7, wherein the PD-1/PD-L1 immune checkpoint blockade therapy is an anti-PD-L1 antibody or an anti-PD-1 antibody.

9. A pharmaceutical composition, which comprises the T cell according to any one of claims 1 to 5 and a pharmaceutically acceptable carrier.

10. A method for isolating a tumor antigen-specific memory CD8⁺ T cells, comprising the following steps:
a. obtaining a single cell suspension from a mouse or human solid tumor draining lymph node;
b. sorting an activated CD62L⁺PD1^{Low/dim}Tim3⁻CD8⁺ T cell from the above single cell suspension,
preferably, for the mouse, sorting a CD44⁺CD62L⁺CD127⁺CD122⁺CCR7⁺PD1^{Low/dim}Tim3⁻ CD39⁻LAG3⁻CD8⁺ T cell from the single cell suspension; for the human, sorting a CD3⁺CD4⁻ CD45RA⁻CD45RO⁺CD62L⁺CD127⁺CD122⁺CCR7⁺PD1^{Low/dim}Tim3⁻CD39⁻ LAG3⁻CD8⁺ T cell from the single cell suspension;
preferably, the T cell is obtained by flow cytometry or magnetic bead sorting; and
c. performing *in vitro* amplification and culture in T cell culture medium containing CD3/28 antibodies, and interleukin 2, interleukin 7 and interleukin 15 at low dose.

11. A method for inhibiting tumor growth, comprising administering the T cell according to any one of claims 1 to 5, wherein the T cell inhibits tumor growth by adoptive cell transfer therapy.

12. The method according to claim 11, wherein the tumor is selected from melanoma and ovarian cancer.

13. The method according to claim 11, further comprising administering a PD-1/PD-L1 immune checkpoint blockade therapy, wherein the PD-1/PD-L1 immune checkpoint blockade therapy is an anti-PD-L1 antibody or an anti-PD-1 antibody.

14. The T cell according to any one of claims 1 to 5, for use in inhibiting tumor growth, wherein the T cell inhibits tumor growth by adoptive cell transfer therapy; preferably, the tumor is selected from melanoma and ovarian cancer.
